# EUROPEAN PATENT SPECIFICATION

(11) **EP 2 910 931 B1**
(45) Date of publication and mention of the grant of the patent: **15.07.2020**
(21) Application number: 13847533.0
(22) Date of filing: 18.10.2013
(51) Int. Cl.: G01N 21/76, C12Q 1/02, G01N 33/18, G01N 33/50

(54) **METHOD FOR QUANTITATIVE DETERMINATION OF BIOSUBSTANCE AND INSTRUMENT FOR QUANTITATIVE DETERMINATION OF BIOSUBSTANCE**
VERFAHREN ZUR QUANTITATIVEN BESTIMMUNG EINER BIOSUBSTANZ UND INSTRUMENT ZUR QUANTITATIVEN BESTIMMUNG EINER BIOSUBSTANZ
PROCÉDÉ POUR LA DÉTERMINATION QUANTITATIVE D'UNE BIOSUBSTANCE ET INSTRUMENT POUR LA DÉTERMINATION QUANTITATIVE D'UNE BIOSUBSTANCE

(30) Priority: 19.10.2012 JP 2012231771
(43) Date of publication of application: 26.08.2015
(73) Proprietor: Hitachi Plant Services Co., Ltd., Tokyo 170-6034 (JP)
(72) Inventor: NAKATSUKA, Yuta, Tokyo 100-8280 (JP); MORI, Shuichi, Tokyo 100-8280 (JP); MIYASHITA, Noe, Tokyo 100-8280 (JP)
(74) Representative: Beetz & Partner mbB
(86) International application number: PCT/JP2013/078335
(87) International publication number: WO 2014/061787

(56) References cited:
- JP-A- 2000 189 197
- JP-A- 2001 136 999
- JP-A- 2003 535 566
- JP-A- 2008 504 841
- JP-A- 2009 136 205
- JP-A- 2013 005 765
- US-A1- 2011 076 706
- VELAZQUEZ MADELINE; FEIRTAG JOELLEN M: "Quenching and enhancement effects of ATP extractants, cleansers, and sanitizers on the detection of the ATP bioluminescence signal", JOURNAL OF FOOD PROTECTION, vol. 60, no. 7, 1 January 1997 (1997-01-01), pages 799-803, XP009189840, ISSN: 0362-028X
- HATTORI N ET AL: "Enhanced microbial biomass assay using mutant luciferase resistant to benzalkonium chloride", ANALYTICAL BIOCHEMISTRY, ACADEMIC PRESS INC, NEW YORK, vol. 319, no. 2, 15 August 2003 (2003-08-15), pages 287-295, XP004437714, ISSN: 0003-2697, DOI: 10.1016/S0003-2697(03)00322-1
- ISHIDA A ET AL: "Enhanced Firefly Bioluminescence Assay of ATP in the Presence of ATP Extractants by Using Diethylaminoethyl-Dextran", ANALYTICAL BIOCHEMISTRY, ACADEMIC PRESS INC, NEW YORK, vol. 305, no. 2, 15 June 2002 (2002-06-15) , pages 236-241, XP027227024, ISSN: 0003-2697 [retrieved on 2002-06-15]

## Description

### BACKGROUND OF THE INVENTION

### TECHNICAL FIELD

The present invention relates to a method for quantifying cellular ATP contained in a sample and a device for quantifying ATP.

### BACKGROUND ART

Examples of a conventionally known method (counting method) for quantifying microbes in a sample include what is called an ATP assay in which the number of microbes is indirectly counted by quantifying ATP (adenosine triphosphate) extracted from microbes. This ATP assay is a method including: causing an ATP extraction reagent to contact microbes contained in a sample; extracting endogenous ATP from the microbes; and counting the number of the microbes in accordance with an amount of luminescence when the ATP is reacted with a luminescent reagent.

The method of ATP assay is also used for hygiene management of water (e.g., see Patent Document 1), and is especially used for measuring microbes in soft drinks (e.g., see Patent Document 2). However, in a case where the number of microbes contained in a sample is counted, optical transparency, color and pH of the sample may affect accuracy of ATP quantification. In this regard, the quantification method in Patent Document 2 is configured to remove quantification inhibitors in the sample by adding a given processing reagent to the sample in advance.

### PRIOR ART DOCUMENTS

### PATENT DOCUMENTS

Patent Document 1: Japanese Patent Application Publication No. 2005-138018
Patent Document 2: Japanese Patent Application Publication No. 2010-11759

US2011/0076706 A1 discloses methods and kits for the rapid detection and/or determination of bacteria in a sample. The methods are based on a bioluminescent assay of ATP following treatment of the bacteria with a lytic agent. In one embodiment, the treatment is preceded by a bioluminescent assay of ATP and the amount of ATP is subtracted from the amount of ATP following the treatment.

Velazquez, M. and Feirtag, J.M., in Journal of Food Protection, 1997, 60(7), 799-803 discloses a study reporting that commonly employed lytic agents (designated ATP extractants) can interfere with a bioluminescent assay for ATP.

### SUMMARY OF THE INVENTION

### TECHNICAL PROBLEMS

However, in a case where the conventional ATP quantification method is applied for measuring ATP amount contained in microbes, the ATP amount contained in the microbes is a minute amount [amol (10⁻¹⁸ mol) level] to inhibit the ATP amount from being accurately quantified in a highly sensitive manner. In addition, since the processing reagent used for preprocessing of the sample also contains a minute amount of ATP, the conventional ATP quantification method fails to accurately quantify the ATP amount in a highly sensitive manner. Further, the conventional ATP quantification method finds it extremely difficult to control density of the processing reagent so as to deal with a quantification range of the ATP amount being at an amol level, inhibiting ATP amount from being accurately quantified in a highly sensitive manner.

Then, it is an object of the present invention to provide a method for quantifying ATP and a device for quantifying ATP that allow ATP of cells contained in a sample to be quantified more accurately in a highly sensitive manner than before.

### SOLUTION TO PROBLEMS

In order to solve the above problems, the present invention provides a method for quantifying ATP of cells contained in a sample, as defined in claim 1.

Further, in order to solve the above problems, the present invention provides a device for quantifying ATP of cells contained in a sample as defined in claim 3.

### ADVANTAGEOUS EFFECTS OF THE INVENTION

The present invention can provide a method for quantifying ATP and a device for quantifying ATP that can quantify ATP of cells contained in a sample more accurately in a highly sensitive manner than before.

### BRIEF DESCRIPTION OF THE DRAWINGS

FIG. 1 illustrates how to construct a device for quantifying ATP according to an embodiment of the present invention;
FIG. 2 is a flowchart outlining a method for quantifying ATP according to the embodiment of the present invention;
FIGS. 3A and 3B are explanatory views of steps of a procedure of the method for quantifying ATP according to an embodiment of the present invention in view of the device for quantifying ATP; and
FIGS. 4A, 4B and 4C are explanatory views of steps of a procedure of the method for quantifying ATP according to a particular embodiment of the present invention in view of the device for quantifying ATP.

### EMBODIMENTS OF THE INVENTION

Next, a description will be given in detail of embodiments of the present invention by referring to the drawings as appropriate.

The method for quantifying ATP and a device for quantifying ATP of the present invention have a main feature in which the ATP contained in cells such as of microbes in a sample is quantified based on a differential amount of luminescence between an amount of luminescence of the ATP contained in cells such as of the microbes in the sample and an amount of luminescence of minute ATP contained in an ATP extractant reagent used for preprocessing the sample.

Hereinafter, the device for quantifying ATP will be described that quantifies ATP (adenosine triphosphate) contained in the microbes in the sample. Then, the method for quantifying ATP of the present invention will be described, with description about operations of the device for quantifying ATP and a principal for quantifying the ATP.

### <Device for quantifying ATP>

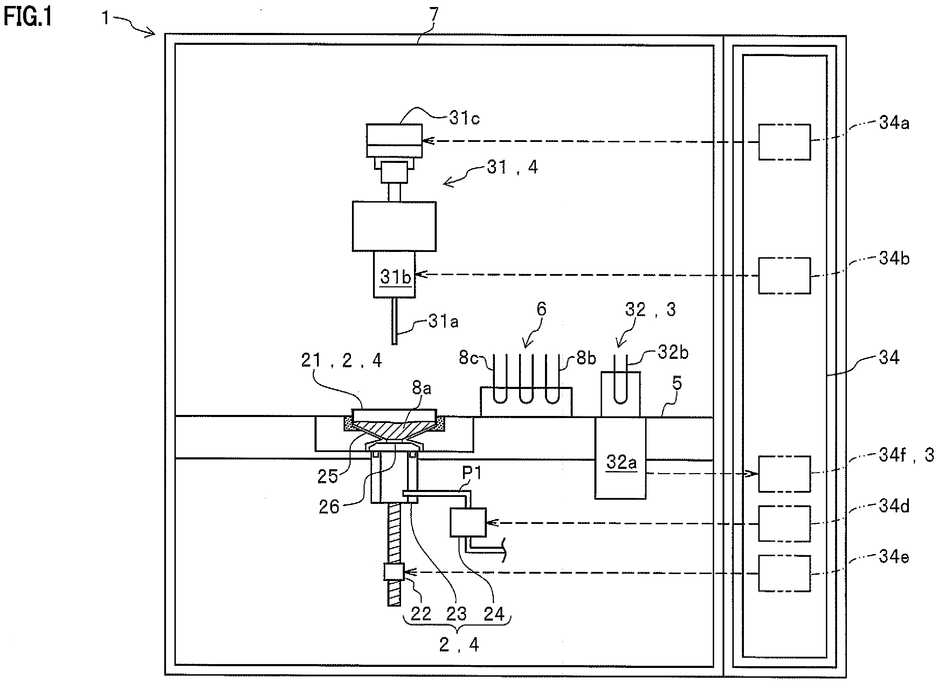

As shown in FIG. 1, a device 1 for quantifying ATP is configured to include: an ATP separation/collection mechanism 4 that separates/collects the ATP contained in the microbes in a liquid sample 8a (sample); and an ATP quantification mechanism 3, each of which is composed of a plurality of elements to be described later.

### (ATP separation/collection mechanism)

The separation/collection mechanism 4 separates/collects the ATP (biological material) from the microbes in the liquid sample 8a.

The separation/collection mechanism 4 includes a collection container 21 having a filter 26 therein, a filtration unit 2 that evacuates contents in the collection container 21 via the filter 26, and a liquid dispensing unit 31 that dispenses a given amount of a reagent and the like.

The collection container 21 separates the microbes contained in the liquid sample 8a that is injected therein, and extracts the ATP in the separated microbes for collection. The separation step of the microbes from the liquid sample 8a and the extraction step of the ATP from the microbes will be described later in detail.

The collection container 21 includes: a funnel-shaped main body 25; and the filter 26 arranged at a bottom of the main body 25. The filter 26 according to the present embodiment has two components in layers although not shown, a hydrophilic filter and a hydrophobic filter. The hydrophilic filter is disposed at the upper side and the hydrophobic filter is disposed at the lower side of the two components.

The filter 26 as so constructed can retain the liquid sample 8a injected into the main body 25 on the filter 26 unless the liquid sample 8a is sucked by a suction head 23 of the filtration unit 2 described later. In addition, when the liquid sample 8a is sucked by the suction head 23, the microbes (not shown) in the liquid sample 8a are retained on the filter 26 and liquid components can be discharged through the filter 26 to the suction head 23 side.

Meanwhile, the collection container 21 is detachably mounted on a predetermined position of a treatment stage 5 arranged in the device 1 for quantifying ATP.

The filtration unit 2 includes: the collection container 21 that also configures the above described separation/collection mechanism 4; the suction head 23; a lift mechanism 22 that moves the suction head 23 up and down; and a suction pump 24 that sucks the contents in the collection container 21 through the suction head 23.

As described above, the suction head 23 is movable up and down via the lift mechanism 22. When the contents in the collection container 21 are filtered by the filter 26, the lift mechanism 22 moves up the suction head 23 so that the suction head 23 is connected with the collection container 21. In addition, the collection container 21 is mounted on the treatment stage 5 or is removed from the treatment stage 5. In these cases, the lift mechanism 22 moves down the suction head 23 to disconnect the suction head 23 from the collection container 21.

Meanwhile, the suction pump 24 is arranged in the middle of a pipe P1 that extends from the suction head 23. When the suction pump 24 is actuated, the liquid components contained in the collection container 21, as described above, pass through the filter 26, the suction head 23, and the suction pump 24 to be discharged into a waste tank (not shown) of the device 1 for quantifying ATP.

In this connection, a suction pump controller 34d and a lift mechanism controller 34e of a control unit 34 can start and/or stop the suction pump 24 and the lift mechanism 22, respectively.

A liquid dispensing unit 31 includes: a liquid dispensing nozzle 31a; a dispensing pump 31b that sucks a predetermined amount of liquid from the liquid dispensing nozzle 31a or discharges that thereto; an actuator 31c that three-dimensionally moves the liquid dispensing nozzle 31a in a housing 7 of the device 1 for quantifying ATP; and an actuator controller 34a and a flow rate controller 34b of the control unit 34.

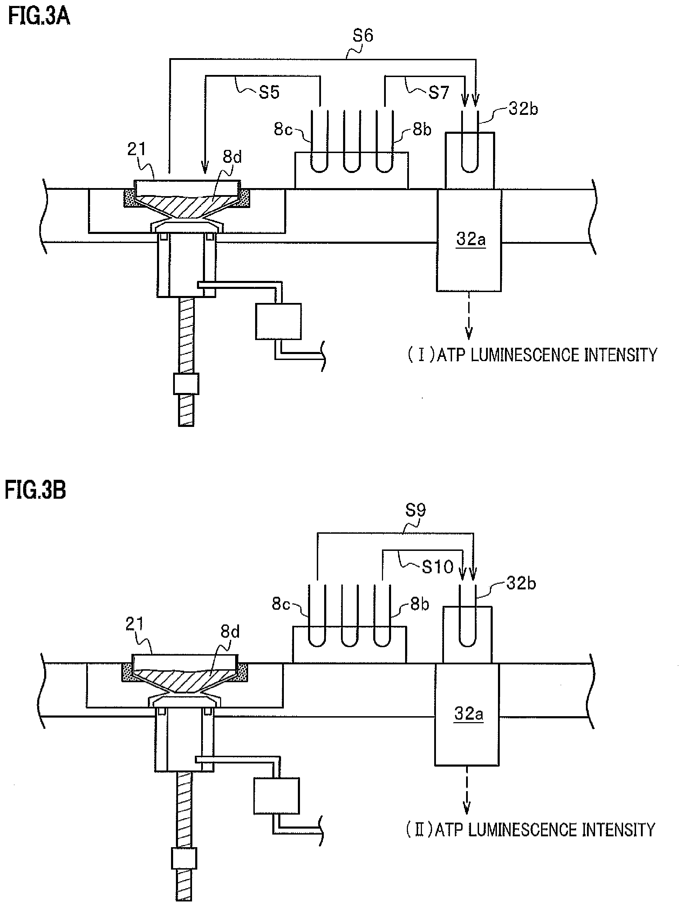

The liquid dispensing nozzle 31a of this liquid dispensing unit 31 is used in a step for measuring ATP luminescence intensity as described below. A predetermined amount of an ATP extract 8d (see FIG. 3A) as obtained in the collection container 21 is dispensed to a tube 32b for luminescence assay performed by an emission intensity-measuring unit 32 included in an ATP-quantification mechanism 3 described later. In addition, the liquid dispensing nozzle 31a dispenses an ATP luminescent reagent 8b placed in a reagent holder 6 to the tube 32b for luminescence assay.

In a case where an ATP amount contained in an ATP extraction reagent 8c, to be described later, as a processing reagent is measured, the liquid dispensing nozzle 31a dispenses the ATP extraction reagent 8c placed in the reagent holder 6 to the tube 32b for luminescence assay. In addition, the liquid dispensing nozzle 31a dispenses the ATP luminescent reagent 8b placed in the reagent holder 6 to the tube 32b for luminescence assay.

The actuator controller 34a controls the actuator 31c in a predetermined manner so that the liquid dispensing nozzle 31a moves three-dimensionally. In addition, the flow rate controller 34b controls the dispensing pump 31b in a predetermined manner so that dispensed amounts of the ATP luminescent reagent 8b, the ATP extraction reagent 8c and the ATP extract 8d (see FIG. 3A) described later, etc., are adjusted.

### (ATP-quantification mechanism)

The ATP-quantification mechanism 3 primarily includes: the emission intensity-measuring unit 32; and an arithmetic part 34f of the control unit 34. The arithmetic part 34f determines quantity of ATP contained in microbes based on detection signals indicating ATP luminescence intensity, outputted from this emission intensity-measuring unit 32.

As described below in detail, the emission intensity-measuring unit 32 includes: the tube 32b for luminescence assay, the tube receiving the dispensed ATP extract 8d (see FIG.3A) or the ATP extraction reagent 8c, and the ATP luminescent reagent 8b to make a luminescent reaction; and a main body 32a for light detection, the main body having a photo multiplier, etc., that detects the ATP luminescence intensity during the luminescent reaction.

Then, the main body 32a for light detection outputs the ATP luminescence intensity detection signals in the tube 32b for light detection to the arithmetic part 34f of the control unit 34. That is, the main body 32a for light detection outputs the detected signals of the "ATP luminescence intensity reflecting an amount of ATP in the ATP extract 8d" and the detected signals of the "ATP luminescence intensity reflecting an amount of ATP in the ATP extraction reagent 8c", respectively.

Note that the "ATP luminescence intensity reflecting an amount of ATP in the ATP extract 8d" corresponds to an "amount of luminescence when an ATP luminescence reagent for ATP is reacted with ATP that is separated/extracted from a cell" in the claims. In addition, the "ATP luminescence intensity reflecting an amount of ATP in the ATP extraction reagent" corresponds to an "amount of luminescence when the ATP luminescence reagent is reacted with the ATP extraction reagent" in the claims.

The arithmetic part 34f calculates differential luminescence intensity between respective ATP luminescence intensities based on the detected signals of the "ATP luminescence intensity reflecting an amount of ATP in the ATP extract 8d" and the detected signals of the "ATP luminescence intensity reflecting an amount of ATP in the ATP extraction reagent". Note that this differential luminescence intensity corresponds to a "differential amount of luminescence" in the claims.

In addition, the arithmetic part 34f calculates ATP amount contained in the microbes in the liquid sample 8a based on this differential luminescence intensity. Note that a procedure of calculating the ATP amount in the arithmetic part 34f will be described later in detail.

### <Operation of device for quantifying ATP and principle of quantifying ATP>

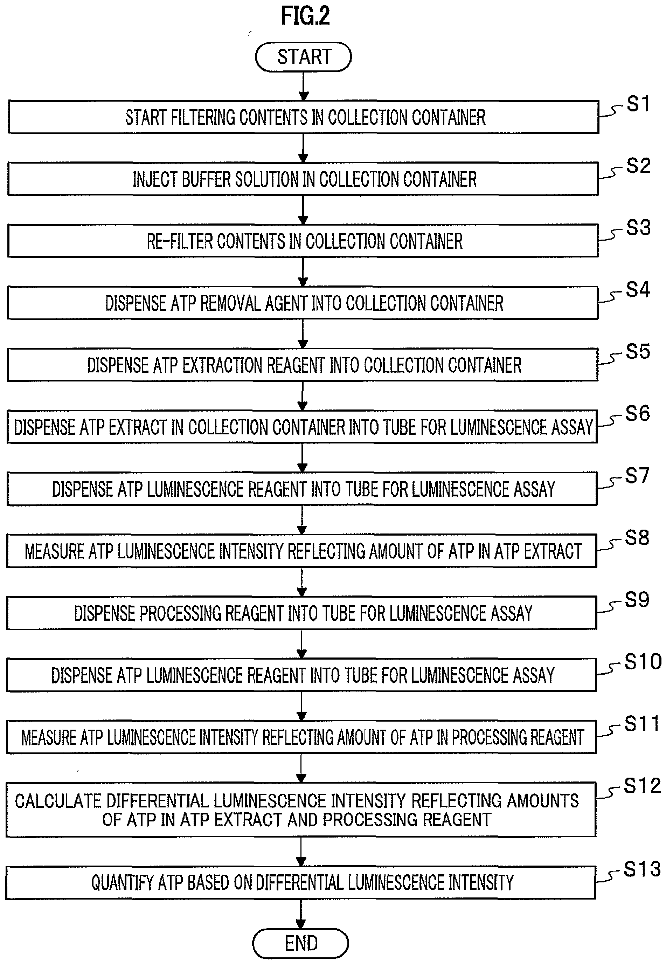

Next, the method for quantifying ATP of the present invention will be described, with description about operations of the device 1 for quantifying ATP and a principle of quantifying ATP. Here, a description will be given with reference to FIGS. 1 and 2. FIG. 2 is a flowchart outlining the method for quantifying ATP according to one embodiment of the present invention.

First, in the device 1 for quantifying ATP as shown in FIG. 1, the collection container 21 is placed on the treatment stage 5. Next, the liquid sample 8a to be a detection target is injected into the collection container 21. Then, a start switch (not shown) is turned on to make the control unit 34 execute the following procedure.

The lift mechanism controller 34e of the control unit 34 commands the lift mechanism 22 so as to lift up the suction head 23 to couple with the collection container 21.

Then, the suction pump controller 34d of the control unit 34 activates the suction pump 24, allowing the suction head 23 to start filtering of the contents (liquid sample 8a) in the collection container 21 (step S1 in FIG. 2). With the filtering step of step S1, the microbes are collected on the filter 26 as well as the liquid components in the liquid sample 8a are discharged toward the suction head 23. On this occasion, most parts of the ATP present outside the cells of microbes contained in the liquid components of the liquid sample 8a and material that may inhibit ATP luminescence reaction later are also discharged with the liquid components.

Upon completion of the filtration of the contents in the collection container 21, a buffer solution is injected into the collection container 21 (step S2 in FIG. 2).

Sterilized hot water or the like without ATP may preferably be used as the buffer solution.

Then, the suction pump controller 34d of the control unit 34 activates the suction pump 24, allowing the suction head 23 to restart the filtration of the contents in the collection container 21 (step S3 in FIG. 2).

In the next step S4, an ATP eliminating reagent is dispensed into the collection container 21 (step S4 in FIG. 2).

Incidentally, the ATP eliminating reagent is placed in the reagent holder 6 and is dispensed from the reagent holder 6 into the collection container 21 through the liquid dispensing nozzle 31a of the liquid dispensing unit 31.

The dispensation of this ATP eliminating reagent completely eliminates the ATP present outside the microbial cells. Examples of this ATP eliminating reagent include an ATPase.

In addition, the liquid dispensing nozzle 31a of the liquid dispensing unit 31 dispenses the ATP extraction reagent 8c into the collection container 21 under the control by the above-mentioned actuator controller 34a and the flow rate controller 34b (step S5 in FIG. 2). Note that the ATP extraction reagent 8c corresponds to a "processing reagent" in Figure 2.

The dispensation of the ATP extraction reagent 8c allows the ATP contained in the microbes to be extracted, and the ATP extract 8d (see FIG. 3A) is produced in the collection container 21. In addition, the ATP eliminating reagent dispensed in the collection container 21 in step S4 is deactivated by contacting with the ATP extraction reagent 8c.

Examples of the ATP extraction reagent 8c that can be suitably used include a surfactant, a mixed solution of ethanol and ammonia, methanol, ethanol, trichloroacetic acid, perchloric acid, and a Tris buffer. Among them, preferred is a surfactant. Examples of the surfactant include sodium dodecyl sulfate, potassium lauryl sulfate, sodium monolauroyl phosphate, sodium alkylbenzene sulfonate, benzalkonium chloride, benzethonium chloride, cetylpyridinium chloride, cetyl trimethyl ammonium bromide, and myristyl dimethyl benzyl ammonium chloride.

In addition, the liquid dispensing nozzle 31a of the liquid dispensing unit 31 dispenses the ATP extract 8d in the collection container 21 into the tube 32b for luminescence assay under the control of the actuator controller 34a and the flow rate controller 34b (step S6 in FIG. 2).

Accordingly, in the tube 32b for luminescence assay, ATP in the ATP extract 8d reacts with the ATP luminescent reagent 8b to emit light.

In addition, the liquid dispensing nozzle 31a dispenses the ATP luminescent reagent 8b placed in the reagent holder 6 into the tube 32b for luminescence assay under the control of the actuator controller 34a and the flow rate controller 34b (step S7 in FIG. 2).

Examples of the ATP luminescent reagent 8b include a luciferase-luciferin reagent.

Next, the arithmetic part 34f of the control unit 34 performs digital processing of detected signals outputted from the main body 32a for light detection after the main body 32a for light detection (see FIG. 1) detects the ATP luminescence intensity, and measures luminescence intensity based on a single-photon counting method (Step S8 in FIG. 2). Note that the luminance intensity corresponds to an "amount of luminescence" in claims as described above. In addition, the step in step S8 corresponds to a "step of measuring a first amount of luminescence" in claims.

Then, the control unit 34 temporarily stores a value (data) of the luminescence intensity measured by the arithmetic part 34f (the amount of luminescence measured in the step of measuring the first amount of luminescence) in a given storage section (not shown).

Next, the tube 32b for luminescence assay in the emission intensity-measuring unit 32 is replaced by a new one. The liquid dispensing nozzle 31a dispenses the ATP extraction reagent 8c (processing reagent) placed in the reagent holder 6 into the tube 32b for luminescence assay under the control of the actuator controller 34a and the flow rate controller 34b (step S9 in FIG. 2).

In addition, the liquid dispensing nozzle 31a dispenses the ATP luminescent reagent 8b placed in the reagent holder 6 into the tube 32b for luminescence assay under the control of the actuator controller 34a and the flow rate controller 34b (step S10 in FIG. 2).

This causes light emitted in this tube 32b for luminescence assay, through luminescent reaction between ATP in the ATP extraction reagent 8c (processing reagent) and the ATP luminescent reagent 8b.

Next, the arithmetic part 34f of the control unit 34 performs digital processing of detected signals outputted from the main body 32a for light detection after the main body 32a for light detection (see FIG. 1) detects the ATP luminescence intensity. The main body 32a for light detection measures the luminescence intensity based on the single photon counting method (Step S11 in FIG. 2). Note that the step in step S11 corresponds to a "step of measuring a second amount of luminescence" in claims.

Then, the control unit 34 temporarily stores a value (data) of the luminescence intensity measured by the arithmetic part 34f (the amount of luminescence measured in the step of measuring the second amount of luminescence) in the storage section.

Incidentally, the step of "measuring the first amount of luminescence" in step S8 and the step of "measuring the second amount of luminescence" in step S11 in the present embodiment are always performed in a pair.

Next, the arithmetic part 34f of the control unit 34 refers to the storage section (not shown) mentioned above to calculate the differential luminance intensity between the luminescence intensity measured in step S8 (the amount of luminescence measured in the step of measuring the first amount of luminescence) and the luminescence intensity measured in step S11 (the amount of luminescence measured in the step of measuring the second amount of luminescence) (step S12 in FIG. 2).

Then, based on a relationship between a pre-stored ATP amount (amol) and luminescence intensity (CPS) which is calculated every time the ATP of a liquid sample is quantified, the control unit 34 calculates an ATP amount (amol) corresponding to the differential luminescence intensity measured above. That is, ATP in the liquid sample 8a (sample) is quantified based on the differential luminescence intensity (step S13 in FIG. 2). This step S13 completes a series of predetermined steps of a method for quantifying ATP according to the present embodiment.

According to the device 1 for quantifying ATP and the method for quantifying ATP, the following advantageous efforts can be obtained. FIGS. 3A and 3B, which will be referred to below, are explanatory views of steps of the procedure of the method for quantifying ATP in the present embodiment in view of the device for quantifying ATP. FIG. 3A shows the procedure in step S5 through step S7 in FIG. 2 and FIG. 3B shows the procedure in step S9 and step S10 in FIG. 2.

As shown in FIG. 3A, the ATP extraction reagent 8c is dispensed into the collection container 21 (step S5 in FIG. 2) to produce the ATP extract 8d in the collection container 21. This ATP extract 8d includes a mixture of the ATP extracted from the microbes in the liquid sample 8a (see FIG. 1) and the ATP contained in the dispensed ATP extraction reagent 8c.

In the next step S6, the ATP extract 8d in the collection container 21 is dispensed into the tube 32b for luminescence assay.

Once the ATP luminescent reagent 8b is dispensed into the tube 32b for luminescence assay (step S7 in FIG. 2), light is emitted in the tube 32b for luminescence assay with the luminescence intensity according to the existing ATP amount. In other words, the main body 32a for light detected outputs the detection signal of the ATP luminescence intensity reflecting a total combined amount of the microbe-derived ATP in the liquid sample 8a and the ATP contained in the dispensed ATP extraction reagent 8c (indicated as (I) ATP luminescence intensity in FIG. 3A).

As shown in FIG. 3B, once the ATP extraction reagent 8c is dispensed into the tube 32b for luminescence assay (step S9 in FIG. 2) and the ATP luminescence reagent 8b is dispensed into the tube 32b for luminescence assay (step S10 in FIG. 2), light is emitted in the tube 32b for luminescence assay with the luminescence intensity according to the ATP amount contained in the dispensed ATP extraction reagent 8c. In other words, the main body 32a for light detection outputs the detected signals of the ATP luminescence intensity reflecting the ATP amount contained in the dispensed ATP extraction reagent 8c (indicated as (II) ATP luminescence intensity in FIG. 3B).

Then, as described above, in step S12 shown in FIG. 2, the differential luminescence intensity is calculated, that is, measurement for calibration is implemented to calculate the ATP luminescence intensity based on only the microbe-derived ATP in the liquid sample 8a is calculated by subtracting the ATP amount contained in the ATP extraction reagent 8c. In addition, in step S13 shown in FIG. 2, the ATP is quantified based on such differential luminescence intensity. In this way, according to the device 1 for quantifying ATP and the method for quantifying ATP of the present embodiment, the microbe-derived ATP amount in the liquid sample 8a can be accurately quantified in a highly sensitive manner.

In addition, as shown in FIGS. 3A and 3B, since the same ATP extraction reagent 8c is used to dispense into the collection container 21 in step S5 and to dispense into the tube 32b for luminescence assay in step S9, not only the density of the ATP extraction reagent 8c itself but also the ATP amount contained in the ATP extraction reagent 8c and density of other unknown inclusions are the same. Further, the same ATP luminescence reagent 8b is used.

Therefore, the device 1 for quantifying ATP and the method for quantifying ATP have no difference to occur in the density of the ATP extraction reagent 8c and of the ATP luminescence reagent 8b, unlike the conventional quantification method. Thus, according to the device 1 for quantifying ATP and the method for quantifying ATP of the present invention, the microbe-derived ATP amount in the liquid sample 8a can be accurately quantified in a highly sensitive manner.

Hereinabove, one embodiment according to the present invention has been described. The present invention, however, is not limited to the above embodiment, and various modifications can be implemented.

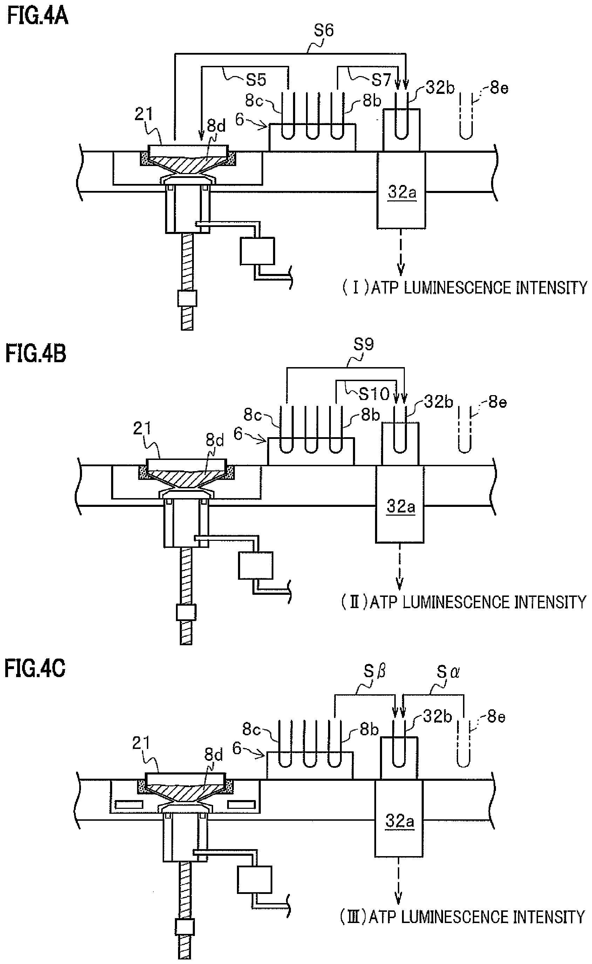

FIGS. 4A, 4B and 4C are explanatory views of steps of a procedure of a method for quantifying ATP in a particular embodiment of the present invention in view of the device for quantifying ATP.

In FIGS. 4A to 4C, the reference numeral 6 indicates the reagent holder, the reference numeral 8d indicates the ATP extract, the reference numeral 8e indicates an ATP standard reagent, the reference numeral 21 indicates the collection container, the reference numeral 32a indicates the main body for light detection, and the reference numeral 32b indicates the tube for luminescence assay.

With the method for quantifying ATP in this embodiment, as described in FIGS. 4A to 4C, the ATP standard reagent 8e having a known ATP amount is separately prepared. The ATP standard reagent 8e can be placed in a housing 7 (see FIG. 1) at a suitable position where the ATP standard reagent 8e can be accessed by the liquid dispensing nozzle 31a (see FIG. 1) of the liquid dispensing unit 31 (see FIG. 1). The ATP standard reagent 8e can, of course, also be placed in the reagent holder 6.

The procedure of the method for quantifying ATP shown in FIGS. 4A and 4B are the same as that shown in FIGS. 3A and 3B. That is, the main body 32a for light detection shown in FIG. 4A outputs the detected signals of the ATP luminescence intensity reflecting the total combined amount of the microbe-derived ATP in the liquid sample 8a and the ATP contained in the dispensed ATP extraction reagent 8c (indicated as (I) ATP luminescence intensity in FIG. 4A). In addition, the main body 32a for light detection shown in FIG. 4B outputs the detected signals of the ATP luminescence intensity reflecting the ATP amount contained in the dispensed ATP extraction reagent 8c (indicated as (II) ATP luminescence intensity in FIG. 4B).

Then, as described above, in step S13 (see FIG. 2) of the embodiment, the control unit 34 calculates the ATP amount (amol) reflecting the differential luminescence intensity based on the calculated relationship between the pre-stored ATP amount (amol) and the luminescence intensity (CPS).

In this particular embodiment, after the procedure shown in FIGS. 4A and 4B of the method for quantifying ATP is performed, the ATP standard reagent 8e, which is separately prepared, is dispensed into the tube 32b for luminescence assay (step Sa) and the same ATP luminescence reagent 8b as used in steps S7 and S10 is dispensed into the tube 32b for luminescence assay (step Sβ). Incidentally, those steps of dispensing the ATP luminescence reagent 8b and the ATP standard reagent 8e are performed under the control of the actuator controller 34a and the flow rate controller 34b shown in FIG. 1.

This causes light emitted in the tube 32b for luminescence assay, through luminescent reaction of the ATP luminescent reagent 8b according to the ATP amount of the dispensed ATP standard reagent 8e. That is, with the ATP luminescence reagent 8b used in the procedure shown in FIGS. 4A and 4B, the main body 32a for light detection outputs the detected signals having the ATP luminescence intensity reflecting the known ATP amount contained in the ATP standard reagent 8e (indicated as (III) ATP luminescence intensity in FIG. 4C).

In this particular embodiment, as in step S12 (see FIG. 2), the differential luminescence intensity is calculated based on the "(I) ATP luminescence intensity" and the "(II) ATP luminescence intensity" shown in FIGS. 4A and 4B, respectively. Then, the ATP reflecting the differential luminescence intensity is quantified based on the "ATP luminescence intensity of the known ATP amount" that is actually measured by "using the above-mentioned ATP luminescence reagent 8b".

According to the particular embodiment, the ATP reflecting the differential luminescence intensity is quantified based on the "ATP luminescence intensity of the known ATP amount" actually measured by "using the same ATP luminescence reagent 8b as used for quantifying the ATP in the microbes". Therefore, the microbe-derived ATP amount in the liquid sample 8a can be more accurately quantified in a highly sensitive manner.

In addition, in the above description, the present invention is configured to measure the ATP luminescence intensity reflecting an amount of the ATP in the ATP extract 8d, and then, to measure the ATP luminescence intensity reflecting an amount of the ATP in the processing reagent. However, the present invention may be configured to measure the ATP luminescence intensity reflecting an amount of the ATP in the processing reagent, and then, to measure the ATP luminescence intensity reflecting an amount of the ATP in the ATP extract 8d.

Also, the device 1 for quantifying ATP may be configured to measure the ATP luminescence intensity reflecting an amount of the ATP in the ATP extract 8d in parallel with the ATP luminescence intensity reflecting an amount of the ATP in the ATP extraction reagent.

Still further, the ATP luminescence reagent 8b is dispensed into the tube 32b for luminescence assay for luminescence reaction after the ATP extract 8d or the ATP extraction reagent 8c (processing reagent) is dispensed. However, in the present invention, the ATP extract 8d or the ATP extraction reagent 8c (processing reagent) may be dispensed into the tube 32b for luminescence assay after the ATP luminescence reagent 8b is dispensed.

Yet further, the ATP standard reagent 8e may be dispensed into the tube 32b for luminescence assay after the ATP luminescence reagent 8b is dispensed.

Examples of the microbes of the above embodiments include, but are not limited to, gram-positive bacteria (e.g., Corynebacteria, Micrococcus, Staphylococcus aureus, Staphylococcus epidermidis, Bacillus cereus, Bacillus subtilis), gram-negative bacteria (e.g., Citrobacter, Escherichia coli, Pseudomonas aeruginosa, Serratia), and fungi (e.g., Aspergillus oryzae, Penicillium notatum, Wallemia, Candida).

Note that when the present invention is applied to spore-forming bacteria such as Bacillus subtilis, the above reagent may further include a cell conversion reagent using nutrients such as amino acids and/or saccharides.

In addition, the above embodiments assume to quantify the ATP in the liquid sample 8a. However, for example, after the gel carrier is used to capture microbes floating in the air, this gel carrier placed in the collection container 21 may be mixed with a buffer solution or the like to prepare the liquid sample 8a.

In this connection, examples of the gel carrier include those made of material that causes a phase transition from gel to sol by raising the temperature from room temperature. Such a material is preferably liquefied in a range from 30°C to less than 40°C. Examples of more preferable material among them include gelatin, a mixture of gelatin and glycerol, and a 10:1 copolymer of N-acryloyl glycinamide and N-methacryloyl-N'-biotinyl propylene diamine.

### EXPLANATION OF REFERENCES

- 1: device for quantifying ATP
- 2: filtration unit
- 4: collection mechanism
- 3: quantification mechanism
- 6: reagent holder
- 8a: liquid sample (sample)
- 8b: ATP luminescence reagent (luminescent reagent for ATP)
- 8c: ATP extraction reagent (processing reagent)
- 8d: ATP extract
- 8e: ATP standard reagent
- 21: collection container
- 22: lift mechanism
- 23: suction head
- 24: suction pump
- 26: filter
- 31: liquid dispensing unit
- 31a: liquid dispensing nozzle
- 31b: dispensing pump
- 31c: actuator
- 32: emission intensity-measuring unit
- 32b: tube for luminescence assay
- 34: control unit
- 34a: actuator controller
- 34b: flow rate controller
- 34d: suction pump controller
- 34e: lift mechanism controller
- 34f: arithmetic part

## Claims

1. A method for quantifying the ATP of microbes contained in a sample, comprising steps of:
measuring a first amount of luminescence when an ATP luminescence reagent is reacted with the ATP that is separated and extracted from the microbes by bringing an ATP extraction reagent into contact with the sample;
measuring a second amount of luminescence that is performed in a pair with the step of measuring the first amount of luminescence when the ATP luminescence reagent is reacted with the ATP extraction reagent and;
measuring a luminescence intensity when the ATP luminescence reagent is reacted with an ATP standard reagent having a known amount of ATP; and
quantifying the ATP of the microbes contained in the sample based on a differential amount of luminescence between the first amount of luminescence and the second amount of luminescence; wherein
the ATP luminescence reagents used in the step of measuring the first amount of luminescence and in the step of measuring the second amount of luminescence belong to the same lot; and
quantifying the ATP based on the differential amount of luminescence comprises calculating an amount of ATP on a basis of the measured luminescence intensity of the standard reagent having a known amount of the ATP.

2. A method for quantifying ATP according to claim 1, wherein the ATP luminescence reagent used in the step of measuring the luminescence intensity of the ATP standard reagent having a known amount of ATP belongs to the same lot as the ATP luminescent reagents used in the step of measuring the first amount of luminescence and the second amount of luminescence.

3. A device for quantifying the ATP of microbes contained in a liquid sample, comprising:
an ATP separation/collection mechanism 4 adapted to separate/collect ATP contained in microbes in a liquid sample;
an ATP quantification mechanism (3), comprising an emission intensity-measuring unit (32), and a control unit (34), an arithmetic part (34f) of which is adapted to determine the quantity of ATP contained in microbes based on detection signals indicating ATP luminescence intensity, outputted from the emission intensity-measuring unit (32);
the control unit (34) being adapted to calculate an amount of ATP of the microbes contained in the sample based on a differential amount of luminescence between an amount of luminescence when an ATP luminescence reagent is reacted with the ATP that is separated and
extracted from the microbes by bringing an ATP extraction reagent into contact with the sample and an amount of luminescence when the ATP luminescence reagent is reacted with the ATP extraction reagent, on a basis of the measured luminescence intensity of a standard reagent having a known amount of ATP.

## Patentansprüche

1. Verfahren zur Quantifizierung des ATP von in einer Probe enthaltenen Mikroben, mit den Schritten:
Messen eines ersten Lumineszenzbetrags, wenn ein ATP-Lumineszenzreagenz mit dem ATP umgesetzt wird, das durch Inkontaktbringen eines ATP-Extraktionsreagenz mit der Probe von den Mikroben abgetrennt und extrahiert wird;
Messen eines zweiten Lumineszenzbetrags, das paarweise mit dem Schritt des Messen des ersten Lumineszenzbetrags durchgeführt wird, wenn das ATP-Lumineszenzreagenz mit dem ATP-Extraktionsreagenz umgesetzt wird, und;
Messen einer Lumineszenzintensität, wenn das ATP-Lumineszenzreagenz mit einem ATP-Standardreagenz umgesetzt wird, das eine bekannte Menge an ATP aufweist; und
Quantifizieren des ATP der in der Probe enthaltenen Mikroben auf der Grundlage eines Differenzbetrags der Lumineszenz zwischen dem ersten Lumineszenzbetrag und dem zweiten Lumineszenzbetrag;
wobei
die ATP-Lumineszenzreagenzien, die im Schritt des Messens des ersten Lumineszenzbetrags und im Schritt des Messens des zweiten Lumineszenzbetrags verwendet werden, zur gleichen Charge gehören; und
das Quantifizieren des ATP auf der Grundlage des Differenzbetrages der Lumineszenz das Berechnen einer ATP-Menge auf der Grundlage der gemessenen Lumineszenzintensität des eine bekannte Menge ATP aufweisenden Standardreagenz umfasst.

2. Verfahren zur Quantifizierung von ATP nach Anspruch 1, bei dem das im Schritt des Messens der Lumineszenzintensität des eine bekannte Menge ATP aufweisenden ATP-Standardreagenz verwendete ATP-Lumineszenzreagenz zur gleichen Charge gehört wie die im Schritt des Messens des ersten Lumineszenzbetrags und des zweiten Lumineszenzbetrags verwendeten ATP-Lumineszenzreagenzien.

3. Vorrichtung zur Quantifizierung des ATP von in einer flüssigen Probe enthaltenen Mikroben, umfassend:
einen ATP-Abtrenn-/Sammelmechanismus (4), der zum Abtrennen/Sammeln von in Mikroben in einer flüssigen Probe enthaltenem ATP ausgelegt ist;
einen ATP-Quantifizierungsmechanismus (3), der eine Emissionsintensitäts-Messeinheit (32) und eine Steuereinheit (34) umfasst, von der ein Arithmetikteil (34f) ausgelegt ist, um die in Mikroben enthaltene ATP-Menge auf der Grundlage von von der Emissionsintensitäts-Messeinheit (32) ausgegebenen, die ATP-Lumineszenzintensität anzeigenden Detektionssignalen zu bestimmen;
wobei die Steuereinheit (34) ausgelegt ist, eine Menge an ATP der in der Probe enthaltenen Mikroben zu berechnen, basierend auf einem Differenzbetrag der Lumineszenz zwischen einem Lumineszenzbetrag, wenn ein ATP-Lumineszenzreagenz mit dem von den Mikroben abgetrennten und extrahierten ATP umgesetzt wird, indem ein ATP-Extraktionsreagenz mit der Probe in Kontakt gebracht wird, und einem Lumineszenzbetrag, wenn das ATP-Lumineszenzreagenz mit dem ATP-Extraktionsreagenz umgesetzt wird, auf der Grundlage der gemessenen Lumineszenzintensität eines eine bekannte Menge ATP aufweisenden Standardreagenz.

## Revendications

1. Méthode de quantification de l'ATP de microbes contenus dans un échantillon, comprenant :
une étape consistant à mesurer une première quantité de luminescence lorsqu'un réactif de luminescence ATP réagit avec l'ATP qui est séparé et extrait des microbes en mettant un réactif d'extraction d'ATP en contact avec l'échantillon ;
une étape consistant à mesurer une deuxième quantité de luminescence lorsque le réactif de luminescence ATP réagit avec le réactif d'extraction d'ATP, qui est effectuée en tandem avec l'étape consistant à mesurer la première quantité de luminescence et ;
une étape consistant à mesurer une intensité de luminescence lorsque le réactif de luminescence ATP réagit avec un réactif ATP standard comportant une quantité connue d'ATP ; et
une étape consistant à quantifier l'ATP des microbes contenus dans l'échantillon sur la base d'une quantité différentielle de luminescence entre la première quantité de luminescence et la deuxième quantité de luminescence ;
dans laquelle
les réactifs de luminescence ATP utilisés dans l'étape de mesure de la première quantité de luminescence et dans l'étape de mesure de la deuxième quantité de luminescence appartiennent au même lot, et
la quantification de l'ATP sur la base de la quantité différentielle de luminescence comprend le calcul d'une quantité d'ATP sur la base de l'intensité de luminescence mesurée du réactif standard comportant une quantité connue d'ATP.

2. Procédé de quantification de l'ATP selon la revendication 1, dans lequel le réactif de luminescence ATP utilisé dans l'étape consistant à mesurer l'intensité de luminescence du réactif ATP standard comportant une quantité connue d'ATP appartient au même lot que les réactifs de luminescence ATP utilisés dans l'étape consistant à mesurer la première quantité de luminescence et la deuxième quantité de luminescence.

3. Dispositif de quantification de l'ATP de microbes contenus dans un échantillon liquide, comprenant :
un mécanisme de séparation/collecte de l'ATP (4) adapté pour séparer/collecter l'ATP contenu dans les microbes dans un échantillon liquide ;
un mécanisme de quantification de l'ATP (3), comprenant une unité de mesure de l'intensité d'émission (32), et une unité de contrôle (34), dont une partie arithmétique (34f) est adaptée pour déterminer la quantité d'ATP contenue dans les microbes sur la base de signaux de détection indiquant l'intensité de luminescence ATP, émis par l'unité de mesure de l'intensité d'émission (32) ;
l'unité de contrôle (34) étant adaptée pour calculer une quantité d'ATP des microbes contenus dans l'échantillon sur la base d'une quantité différentielle de luminescence entre une quantité de luminescence lorsqu'un réactif de luminescence ATP réagit avec l'ATP qui est séparé et extrait des microbes en mettant un réactif d'extraction ATP en contact avec l'échantillon, et une quantité de luminescence lorsque le réactif de luminescence ATP réagit avec le réactif d'extraction ATP, sur la base de l'intensité de luminescence mesurée d'un réactif standard comportant une quantité connue d'ATP.
